# EUROPÄISCHE PATENTSCHRIFT

(11) **EP 1 409 513 B1**
(45) Veröffentlichungstag und Bekanntmachung des Hinweises auf die Patenterteilung: **12.04.2006**
(21) Anmeldenummer: 02779279.5
(22) Anmeldetag: 28.08.2002
(51) Int. Cl.: C07J 73/00, A61K 31/58, A61P 5/28

(54) **METHYLEN-4-AZASTEROIDE**
METHYLENE-4-AZASTEROIDS
METHYLENE-4-AZASTEROIDES

(30) Priorität: 28.08.2001 DE 10141984
(43) Veröffentlichungstag der Anmeldung: 21.04.2004
(73) Patentinhaber: Schering AG, 13353 Berlin (DE)
(72) Erfinder: MENZENBACH, Bernd, 07743 Jena (DE); DROESCHER, Peter, 99425 Weimar (DE); HILLISCH, Alexander, 07743 Jena (DE); ELGER, Walter, 14195 Berlin 33 (DE); SCHWEIKERT, Hans-Udo, 53125 Bonn (DE); SCHÖLLKOPF, Klaus, 13467 Berlin (DE)
(74) Vertreter: Cramer, Eva-Maria
(86) Internationale Anmeldenummer: PCT/EP2002/009587
(87) Internationale Veröffentlichungsnummer: WO 2003/020744

(56) Entgegenhaltungen:
- WO-A-02/00681
- WO-A-02/19971
- WO-A-93/15104
- WO-A-97/15558
- WO-A-98/33506
- RASMUSSON G H ET AL: "AZASTEROIDS AS INHIBITORS OF RAT PROSTATIC 5ALPHA-REDUCTASE" JOURNAL OF MEDICINAL CHEMISTRY, AMERICAN CHEMICAL SOCIETY. WASHINGTON, US, Bd. 27, Nr. 12, 1. Dezember 1984 (1984-12-01), Seiten 1690-1701, XP002043194 ISSN: 0022-2623
- BROOKS J R ET AL: "5ALPHA-REDUCTASE INHIBITORY AND ANTI-ANDROGENIC ACTIVITIES OF SOME 4-AZASTEROIDS IN THE RAT" STEROIDS: STRUCTURE, FUNCTION, AND REGULATION, ELSEVIER SCIENCE PUBLISHERS, NEW YORK, NY, US, Bd. 47, Nr. 1, 1986, Seiten 1-19, XP000611237 ISSN: 0039-128X
- RASMUSSON G H ET AL: "AZASTEROIDS: STRUCTURE-ACTIVITY RELATIONSHIPS FOR INHIBITION OF 5ALPHA-REDUCTASE AND OF ANDROGEN RECEPTOR BINDING" JOURNAL OF MEDICINAL CHEMISTRY, AMERICAN CHEMICAL SOCIETY. WASHINGTON, US, Bd. 29, Nr. 11, 1. November 1986 (1986-11-01), Seiten 2298-2315, XP000568779 ISSN: 0022-2623

## Beschreibung

Die Erfindung betrifft neue 17-Methylen-4-azasteroide der allgemeinen Formel I 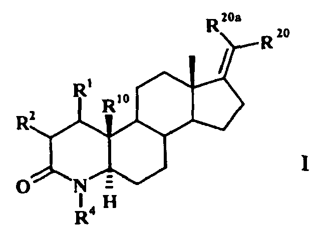 worin
R²⁰ und R^{20a} getrennt Fluoro-, Chloro-, Bromo-, Cyanogruppen Azido, Rhodano, C₁₋₄ Alkyl oder Hydroxy C₁₋₄ Alkyl darstellen, und R^{20a} weiter ein Wasserstoffatom darstellen kann.
R¹⁰ steht jeweils für ein Wasserstoffatom oder eine Methylgruppe, und R⁴ steht für ein Wasserstoffatom oder C₁₋₄ Alkyl.
R¹ und R² sind gemeinsam jeweils ein Wasserstoffatom oder eine zusätzliche Bindung.
Es werden Verfahren zur Herstellung der neuen Verbindungen beschrieben sowie zur Herstellung ihrer pharmazeutischen Zusammensetzungen.
Die erfindungsgemäßen Verbindungen -17-Methylen-4-azasteroide- sind neu, ihre Darstellung und ihre biologische Wirksamkeit wurden bisher noch nicht beschrieben.
Erfindungsgemäß bevorzugte Verbindungen sind in Anspruch 3 angeführt. Gegenstand der vorliegenden Erfindung sind ferner pharmazeutische Zusammensetzungen, die als Wirkstoffe mindestens ein 17- Methylen-4-azasteroid der allgemeinen Formel I enthalten, wobei diese Zusammensetzungen gegebenenfalls geeignete Hilfs- und Trägerstoffe beinhalten. WO-A-97/15558 beschreibt entsprechende 7β-methyl-substituierte Steroide zur Hemmung von 5α-Reduktase.
Die erfindungsgemäßen Verbindungen sind Inhibitoren der 5α-Reduktase. Sie eignen sich daher zur Behandlung von Erkrankungen, die Folge erhöhter Testosteron und letztlich Dihydrotestosteronspiegel in Blut und Gewebe sind.
Erkrankungen durch exzessive Androgeneffekte können auch bei normalen Testosteronspiegeln im Blut auftreten wenn im Gewebe die umwandlung von Testosteron zu Dihydrotestosteron erhöht ist. Dies ist beispielsweise bei idiopathischen Formen des Hirsutismus der Fall (REF).
Progesteron spielt eine wichtige Rolle für den festen Verschluss des Muttermundes (Mahendroo). Dessen Erweichung vor der Geburt resultiert aus einem lokalen Abbau von Progesteron durch 5α-Reduktase zu Dihydroprogesteron, das ein sehr schwaches Gestagen ist. Durch die Hemmung des Katabolismus von Progesteron in diesem Teil des Uterus sind die erfindungsgemäßen Substanzen daher auch geeignet eine vorzeitige Reifung und Eröffnung des Muttermundes zu verhindern.
5α-reduzierte Metabolite von Progesteron (REF aus Lancet) und anderen C21-Steroiden und deren im Körper entstehenden Metabolite, zum Beispiel allo-Pregnanolon, können als Neurosteroide wirken und mit Neurosteroiden interagieren. Störungen dieser Funktionen können sich in Beeinträchtigungen der Befindlichkeit niederschlagen.. Mögliche Indikationen für die erfindungsgemäßen Substanzen sind Prostataerkrankungen, Alopezie vom männlichen Typ und Akne und Hirsutismus, sowie verschiedene gynäkologische Kranheitsbilder wie das prämenstruelle Syndrom. Bei dessen Entstehung spielt das Auftreten von 5α-reduzierten Metaboliten des Progesterons im ZNS eine wichtige RolleDie vorzeitige Eröffnung des Muttermundes kann durch den verstärkten Abbau von Progesteron zu in diesem Gewebe zu Dihydroprogesteron induziert werden. Die erfindungsgemäßen Substanzen sind geeignet diesen Katabolismus und damit die vorzeitige Reifung der Cervix uteri zu verhindern. Die erfindungsgemäßen Substanzen können ihre Wirkung durch die Hemmung der 5α-Reduktion von Testosteron oder Progesteron in den von der Störung betroffenen Organen und Geweben ausüben. Hinzu tritt die Senkung der Blutspiegel der entsprechenden 5α-reduzierten Metabolite.

Die erfindungsgemäßen Verbindungen stellen ferner Zwischenprodukte zur Synthese weiterer pharmakologisch hochwirksamer Steroidprodukte dar.
Die Herstellung der erfindungsgemäßen Verbindungen erfolgt nach den Ansprüchen 4 und 5.

Die Verbindungen nach Anspruch 1 sind erfindungsgemäß aus den 17-Methylensteroiden der allgemeinen Formel II und der allgemeinen Formel VII zugänglich.

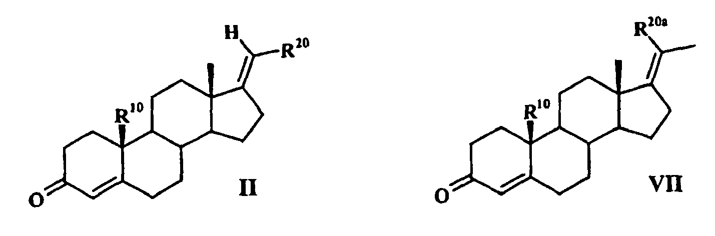

Es werden Verbindungen nach Anspruch 1 erhalten, indem man Verbindungen der allgemeinen Formel II in an sich bekannter Weise mit NalO₄ unter katalytischer Vermittlung von KMnO₄ in einem protischen Lösungsmittel, vorzugsweise t.-BuOH zur 3,5-Seco-ketosäure umsetzt, diese mit NH₄OAc in Eisessig zum ungesättigten Lactam cyclisiert und mit HCOOH/ K₂CO₃ in DMF zum gesättigten Lactam der allgemeinen Formel III reduziert, 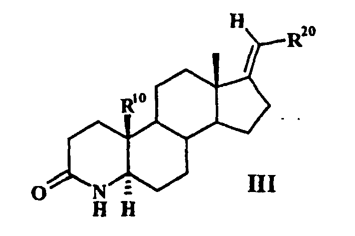 weiterhin die Verbindungen der allgemeinen Formel III mit Mel und NaH in einem dipolaren aprotischen Lösungsmittel, vorzugsweise DMF zu den 4-methylsubstituierten Verbindungen der allgemeinen Formel IV umsetzt, 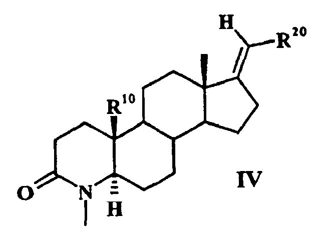 sowie zum anderen die Verbindungen der allgemeinen Formel III in einer silylvermittelten DDQ(2,3-Dichlor-5,6-dicyano-1,4-benzochinon)-Oxidation in einem dipolaren aprotischen Lösungsmittel, wie Dioxan zu den dehydrierten Lactamen der allgemeinen Formel V führt.

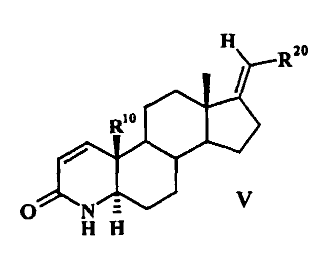

Die Verbindungen der allgemeinen Formel V werden analog den Verbindungen der allgemeinen Formel III mit Mel und NaH zu den 4-methylsubstituierten Lactamen der allgemeinen Formel VI umgesetzt. 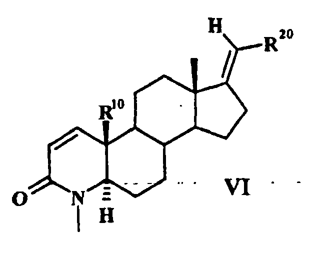 Es werden weiterhin Verbindungen nach Anspruch 1 erhalten, indem man Verbindungen der allgemeinen Formel VII der gleichen Chemie unterwirft wie die Verbindungen der allgemeinen Formel II und dann Verbindungen der allgemeinen Formeln VIII, IX, X und XI erhält.

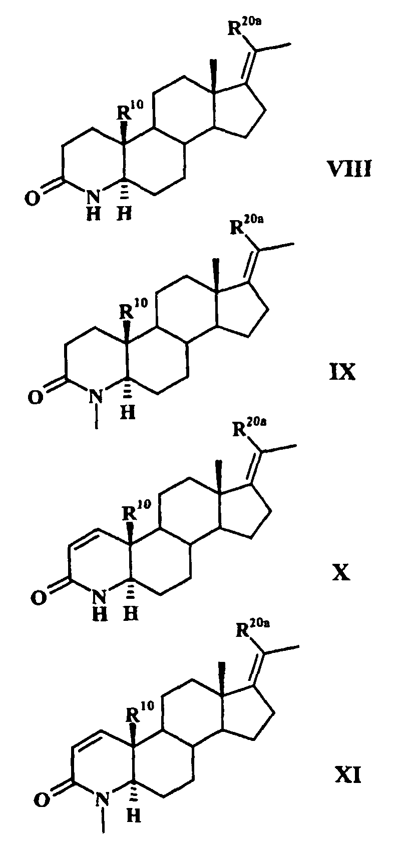

### Ergebnisse

Auswertung der antiandrogenen Aktivität in der kastrierten männlichen Ratte (s. Abbildungen/ Tabelle). Kastrierte immature männliche Ratten wurden über 7 Tage mit Testosteronproprionat (TP) und den erfindungsgemäßen Substanzen (0.1mg TP allein und oder 1mg 5α-Reduktasehemmer/Tier/Tag s.c., n=5-10/Gruppe) behandelt. Die Behandlung mit TP führte zu einer starken Erhöhung der Gewichte der azessorischen Geschlechtsdrüsen (Prostata und Samenblase). Bei mit dem Vehikel und mit 5α-Reduktasehemmer allein behandelten Tieren verharren die Gewichte der untersuchten Organe bei niedrigen Werten. Es wurde gefunden, dass die erfindungsgemäßen Substanzen in den ausgewählten Testsystemen Hemmeffekte ausübten.
Tabelle XX zeigt, dass die Verbindungen J 1879 und J 1924 die Effekte des TP auf Prostata und Samenblase deutlich abschwächen.

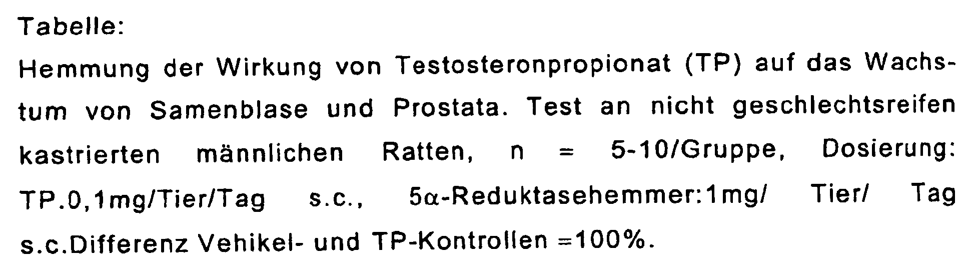

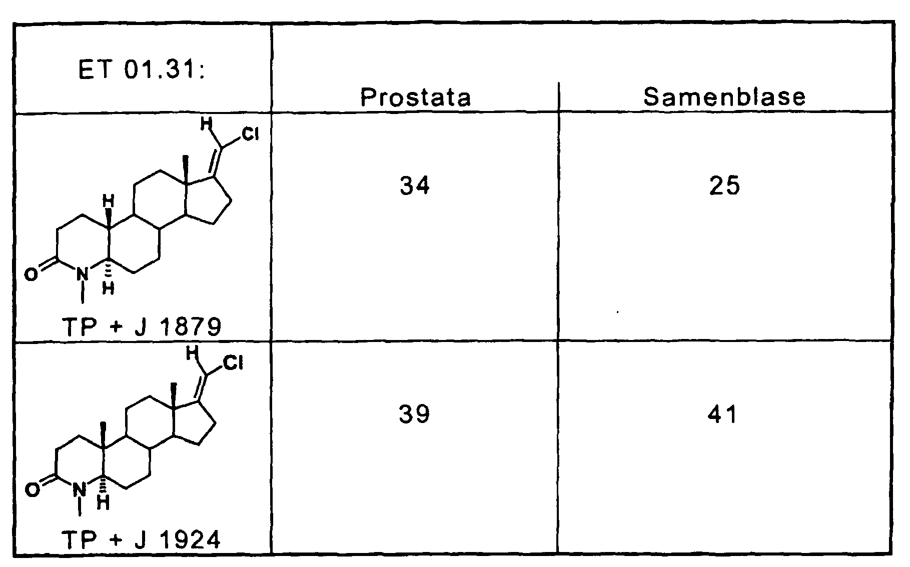

Die Untersuchungen zur Bestimmung der IC₅₀-Werte für die 5α-Reduktase durch die Steroide J1879 und J1924 außerhalb des Genitaltraktes wurden in 4 verschiedenen menschlichen Knochenzellinien (hOB-Zellen) durchgeführt. Die Zellen wurden während 6 Stunden jeweils mit 0,5 µM Androstendion (0,1 µM [³H] Androstendion, 0,4 µM Androstendion) mit oder ohne den Zusatz ansteigender Konzentrationen der Hemmer (10⁻¹¹ - 10⁻⁸ M) inkubiert.

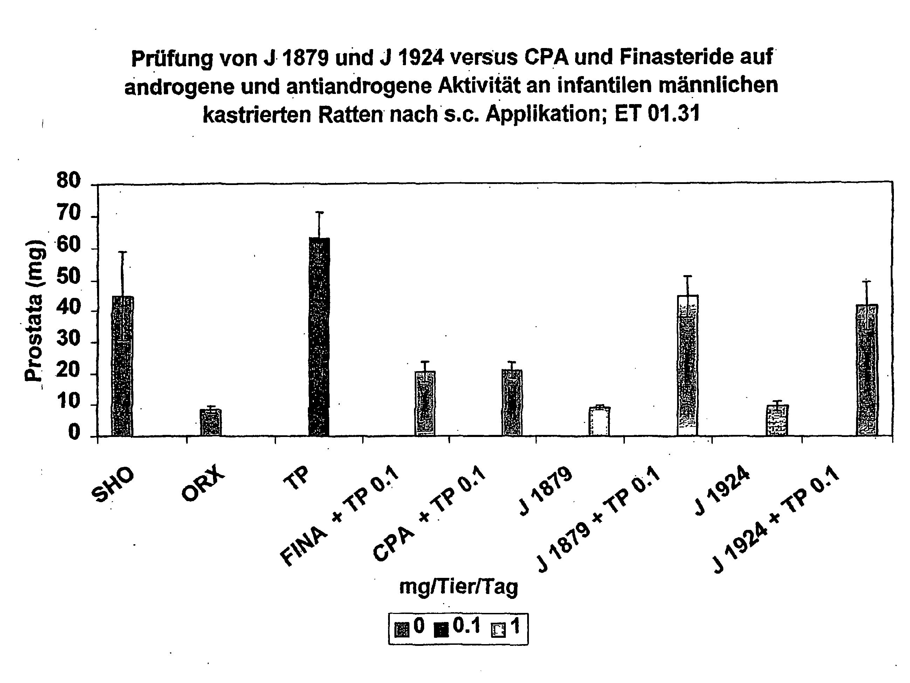

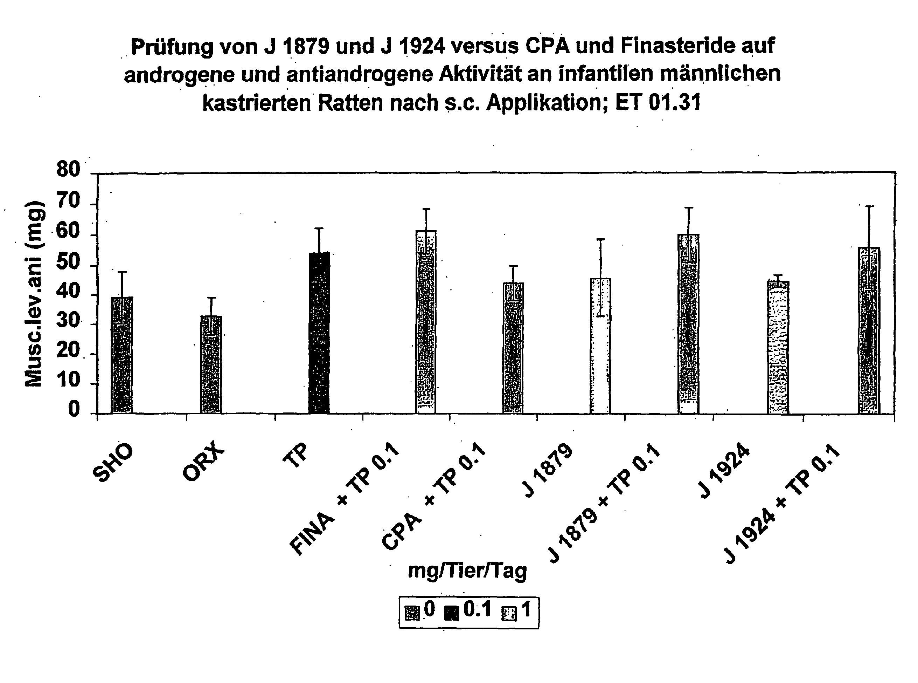

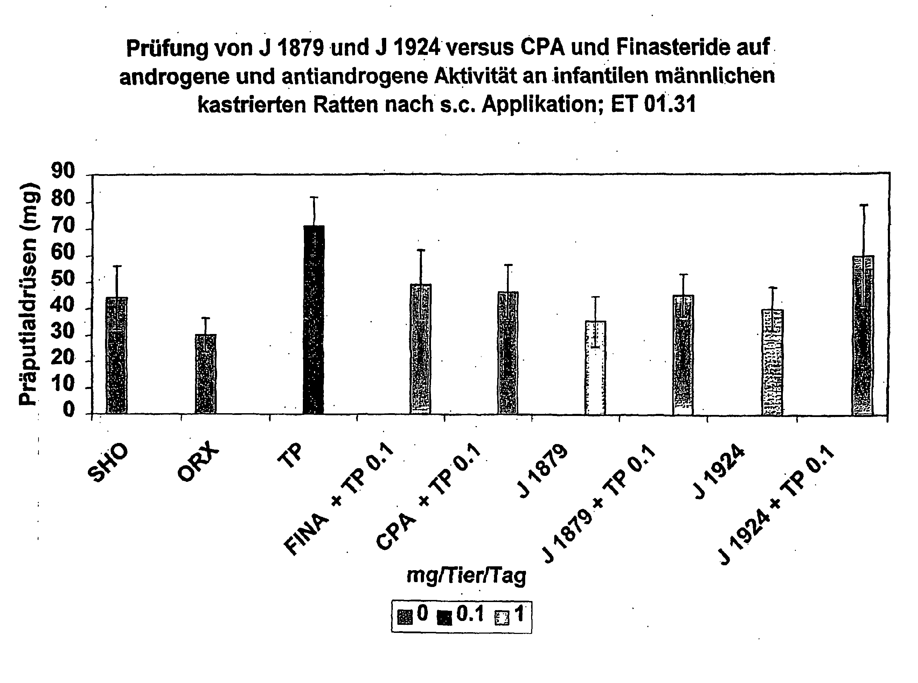

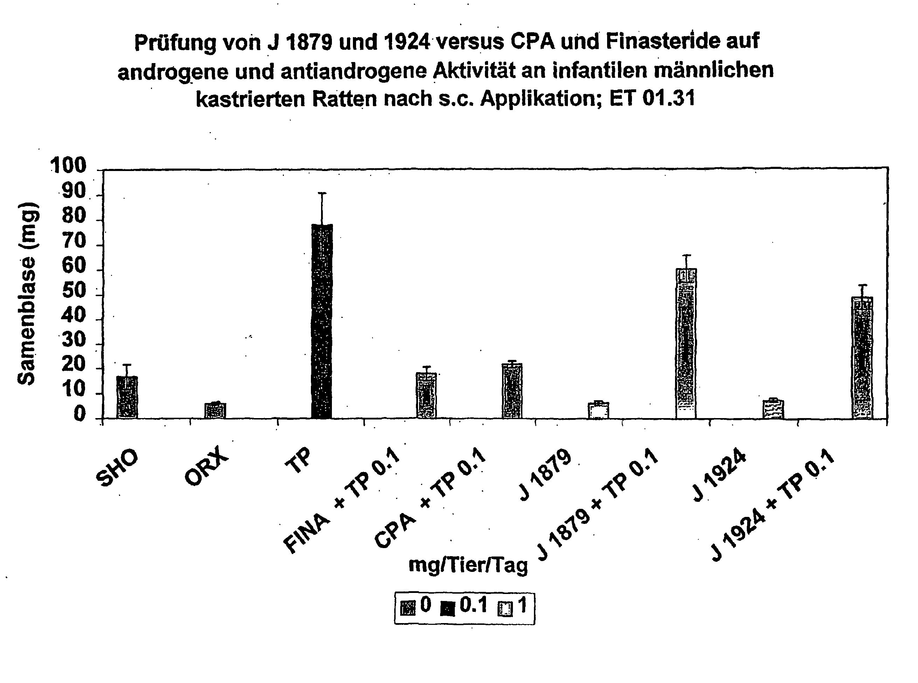

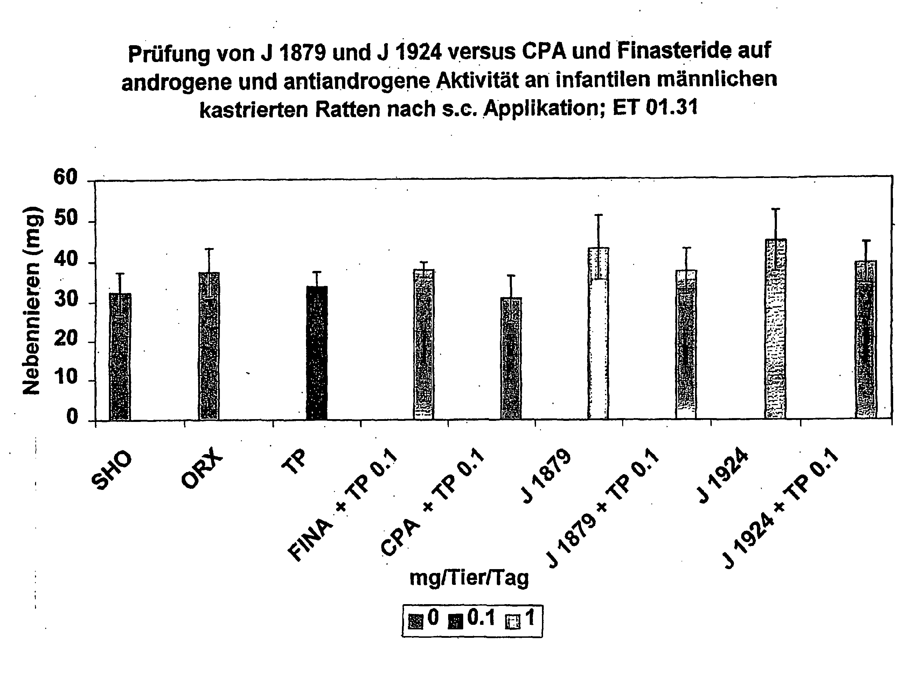

Nach der Inkubation wurde das Medium mit Chloroform : Methanol (2:1; vol:vol) extrahiert und die Steroide (Substrat und 5α-reduzierte Metaboliten) dünnschicht-chromatographisch getrennt und der DNA-Gehalt der Zellen der Proben bestimmt. Die 5α-Reduktaseaktivität (Summe der gebildeten 5α-reduzierten Metabolite: 5α-Androstandion, 5α-Dihydrotestosteron, 5α-Androstan-3α,17β-diol, 5α-Androstan-3β,17β-diol ausgedrückt in pmol / µg DNA / h) wurde jeweils in Duplikatproben ermittelt. Die relative 5α-Reduktaseaktivität (5α-Reduktaseaktivität der Proben mit Zusatz der Hemmer im Vergleich zu den entsprechenden Werten der Kontrollen, d.h. den Proben ohne Zusatz von Hemmern) ist in der Abbildung jeweils als Mittelwert ± SEM aufgetragen. Die IC₅₀ Werte von J1879 und J1924 für die 5α-Reduktase in Knochenzellen betragen jeweils < 10⁻¹⁰ M.

Im Rahmen der Untersuchungen zur Ermittlung der IC₅₀-Werte von J1879 und J 1924 wurde für Vergleichszwecke zusätzlich die Hemmwirkung von LY191704, einem nichtsteroidalen spezifischen Hemmer der 5α-Reduktase Typ 1, Finasteride einem 4-Azasteroid mit vorwiegend hemmender Wirkung auf die 5α-Reduktase Typ 2 und Progesteron einem physiologischen Substrat der 5α-Reduktase in allen 4 Zellinien bei einer Konzentration von jeweils 10⁻⁸ M bestimmt. Aus Abbildung ergibt sich, dass die erfindungsgemäßen Substanzen die 5α-Reduktase in den untersuchten Zelltypen stärker und in wesentlich niedrigeren Konzentrationen hemmen als die genannten natürlichen Substrate und Referenzsubstanzen.

Die Erfindung wird durch die nachfolgenden Beispiele näher erläutert.

### Beispiel 1

### E.17-Chlormethylen-4-aza-5α-androstan-3on

Zu einer Lösung von 3,1 mmol(1,0g) E-17-Chlormethylen-4-aza-androst-5-en-3-on in 140 ml Dimethylformamid werden 59 ml Ameisensäure (85% ig) und 115,8 mmol (16,g) Kalium-karbonat unter Rühren zugegeben und die Reaktionsmischung unter Rückfluß 8 Stunden erhitzt. Anschließend wird die Reaktionslösung mit Toluen versetzt und im Vakuum eingeengt. Der Rückstand wird in Wasser aufgenommen und mit Methylenchlorid extrahiert. Die vereinigten Extrakte werden mit gesättigter Natriumkarbonatlösung behandelt, mit Wasser neutralgewaschen, über Natriumsulfat getrocknet und eingeengt.

Das anfallende Rohprodukt wird aus Aceton / n-Hexan umkristallisiert und man erhält 543 mg festes Produkt (54 %)
Fp=148 - 151°C; [α]_{D}²⁰ =+16° (CHCl₃)

### Beispiel 2

### E-17-Chlormethylen-4-methyl-4-aza-5α-androstan -3-on

Zu einer Suspension von 1,06 mmol (340 mg) E-17-Chlormethylen-4-aza-5α-androstan -3-on in 9 ml Dimethylformamid werden bei Raumtemperatur und Argonatmosphäre 3,08 mmol (123mg) Natrium -hydrid (60 % ig in Öl) zugegeben. Das Reaktionsgemisch wird 30 Minuten gerührt und anschließend eine Lösung aus 5,3 mmol (0,33 ml) Methyljodid in 3,0 ml Dimethylformamid zugetropft. Nach ca. 60 Minuten werden 2 ml Methanol und nach weiteren 10 Minuten 9 ml gesättigte wässrige Ammonium-chloridlösung zugegeben. Das Reaktionsgemisch wird mit Wasser verdünnt und mit Toluen extrahiert. Die vereinigten Extrakte werden mit Wasser gewaschen, über Natriumsulfat getrocknet und eingeengt. Das anfallende Rohprodukt wird aus Aceton / n-Hexan umkristallisiert und man erhält 154 mg festes Produkt (43 %)
Fp=150-162 °C; [α]_{D}²⁰ = -7° (CHCl₃)

### Beispiel 3

### E-17-Chlormethylen-4-aza-5α-androst-1-en-3-on

1,34 mmol (430 mg) E-17-Chlormethylen-4-aza-5α-androstan -3-on werden unter Argon-atmosphäre bei Raumtemperatur in 9 ml Dioxan suspendiert und nachfolgend 1,5 mmol (340 mg) 2,3-Dichlor-5,6-dicyano-p-benzochinon sowie 6,4 mmol (1,7 ml) Bis-(trimethylsilyl)-trifluor-acetamid zugegeben. Es wird zunächst 3 Stunden bei Raumtemperatur und anschließend 3 Stunden im Ölbad bei ca. 100-110 °C gerührt. Die Reaktionslösung wird mit Methylenchlorid verdünnt und nacheinander mit 2% iger wässriger Natriumhydrogensulfitlösung, 2N Salzsäure und Wasser gewaschen. Der verbleibende Extrakt wird über Natriumsulfat getrocknet und eingeengt. Nach Umkristallisation aus Aceton erhält man 243 mg festes Produkt (57 %)
Fp=275-282 °C; [α]_{D}²⁰ = -41° (CHCl₃)

### Beispiel 4

### E-17-Chloromethylen-4-methyl-4-aza-5α-androst-1-en-3-on

Zu einer Suspension von 0,87 mmol (279 mg) E-17-Chlormethylen-4-aza-5α-androst-1-en-3-on in 7 ml Dimethylformamid werden bei Raumtemperatur und Argonatmosphäre 2,4 mmol (98 mg) Natri- umhydrid (60 % ig in Öl;) zugegeben. Das Reaktionsgemisch wird 30 Minuten gerührt und anschließend eine Lösung aus 7,5 mmol (0,47 ml) Methyljodid in 3 ml Dimethylformad zugetropft. Nach ca. 60 Minuten werden 2 ml Methanol und nach weiteren 10 Minuten 9 ml gesättigte wässrige Ammoniumchloridlösung zugegeben. Nach Verdünnung des Reaktionsgemisches mit Wasser wird mit Toluen extrahiert, der Extrakt mit Wasser gewaschen, über Natriumsulfat getrocknet und eingeengt. Das anfallende Rohprodukt wird aus Essigester umkristallisiert und man erhält 122 mg festes Produkt (42 %)
Fp=160-165°C; [α]_{D}²⁰ =-47° (CHCl₃)

### Beispiel 5

### E-17-Chlormethylen-4-aza-5α-estran-3-on

Zu einer Suspension von 3,27 mmol (1g) E-17-Chlormethylen-4-aza-estr-5-en-3-on in 150 ml Dimethylformamid werden 73,3 ml Ameisensäure (85% ig) und 121,55 mmol (16,8g) Kaliumkarbonat unter Rühren zugegeben und das Reaktionsgemisch unter Rückfluß 12 Stunden erhitzt. Anschließend wird die Reaktionlösung mit Toluen versetzt und im Vakuum eingeengt. Der Rückstand wird in Wasser aufgenommen und mit Methylenchlorid extrahiert. Die vereinigten Extrakte werden mit gesättigter Natriumkarbonatlösung behandelt, mit Wasser neutralgewaschen, über Natriumsulfat getrocknet und eingeengt.
Das anfallende Rohprodukt wird aus Aceton / n-Hexan umkristallisiert und man erhält 461 mg festes Produkt (46 %)
Fp= (178-200) 262-82 °C; [α]_{D}²⁰ = -21 °(CHCl₃)

### Beispiel 6

### E-17-Chlormethylen-4-methyl-4-aza-5α-estran-3-on

Zu einer Suspension von 0,65 mmol (200 mg) E-17-Chlormethylen-4-aza-5α-estran-3-on in 5,8 ml Dimethylformamid werden bei Raumtemperatur und Argonatmosphäre 1,7 mmol (73 mg) Natriumhydrid (60 % ig in Öl;) gegeben. Das Reaktionsgemisch wird 30 Minuten gerührt und anschließend eine Lösung aus 3.2 mmol (0,2 ml) Methyljodid in 2 ml Dimethylformamid zugetropft. Nach ca. 60 Minuten werden 1 ml Methanol und nach weiteren 10 Minuten 4 ml gesättigte wässrige Ammoniumchloridlösung zugegeben. Das Reaktionsgemisch wird mit Wasser verdünnt und mit Methylenchlorid extrahiert. Die vereinigten Extrakte werden mit Wasser gewaschen über Natriumsulfat getrocknet und eingeengt. Das anfallende Rohprodukt wird durch Chromatographie an Kieselgel 60 (Eluent Methylenchlorid / Aceton = 8/2) gereinigt und man erhält nach Umkristallisation aus Ethylacetat 131 mg festes Produkt (62 %)
Fp = 161-171°C; [α]_{D}²⁰ = -88° (CHCl₃)

### Beispiel 7

### E-17-Chlormethylen-4-aza-5α-estr-1-en-3-on

1 mmol (310 mg) E-17-Chlormethylen-4-aza-5α-estran-3-on werden unter Argonatmosphäre bei Raumtemperatur in 6,3 ml Dioxan suspendiert und nachfolgend 2,3 mmol (522 mg) 2,3 Dichlor-5,6-dicyan-p-benzochinon sowie 9,8 mmol (2,6 ml) Bis-(trimethylsilyl)-trifluoracetamid zugegeben. Es wird zunächst bei Raumtemperatur 3 Stunden und anschließend 15 Stunden im Ölbad bei 100-110 °C gerührt. Die Reaktionslösung wird mit Methylenchlorid verdünnt und nacheinander mit 2% iger wässriger Natriumhydrogensulfitlösung, 2N Salzsäure und Wasser gewaschen, Der verbleibende Extrakt wird über Natriumsulfat getrocknet und eingeengt. Nach Reinigung durch Chromatographie an Kieselgel 60 (Eluent Methylenchlorid/ Methanol = 98/2) erhält man 57 mg festes Produkt (18,5 %)
[α]_{D}²⁰ = 37° (CHCl₃)

## Patentansprüche

1. Halogen- und pseudohalogensubstituierte 17-Methylen-4-azasteroide der allgemeinen Formel I, worin 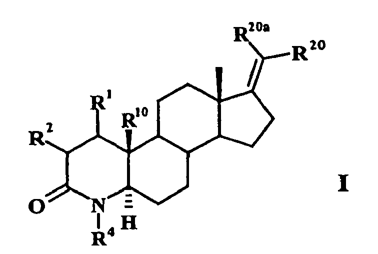
R²⁰ und R^{20a} getrennt Fluoro-, Chloro-, Bromo-, Azido-, Cyano, Rhodano-, C₁₋₄-Alkyl oder Hydroxy-C₁₋₄-alkyl darstellen, und R^{20a} weiter ein Wasserstoffatom darstellen kann
R¹⁰ für ein Wasserstoffatom oder eine Methylgruppe steht,
R⁴ ausgewählt aus einer Gruppe, bestehend aus Wasserstoff und C₁₋₄-Alkyl, ist,
R¹ und R² gemeinsam jeweils ein Wasserstoffatom oder eine zusätzliche Bindung sind.

2. Halogen- und pseudohalogensubstituierte 17-Methylen-4-azasteroide nach Anspruch 1,
worin
R⁴ = H, Me
R²⁰ und R^{20a} getrennt = F, Cl, Br
bedeutet.

3. 17-Methylen-4-azasteroide nach Anspruch 1 ,
nämlich
E-17-Chlormethylen-4-aza-5α-estran-3-on
E-17-Chlormethylen-4-aza-5α-androstan-3-on
E-17-Chlormethylen-4-methyl-4-aza-5α-estran-3-on
E-17-Chlormethylen-4-methyl-4-aza-5α-androstan-3-on
E-17-Chlormethylen-4-aza-5α-estr-1-en-3-on
E-17-Chlormethylen-4-aza-5α-androst-1-en-3-on
E-17-Chlormethylen-4-methyl-4-aza-5α-androst-1-en-3-on
E-17-Brommethylen-4-methyl-4-aza-5α-estran-3-on
E-17-Cyanomethylen-4-methyl-4-aza-5α-estran-3-on
Z-17-(1')-Chlorethyliden-4-methyl-4-aza-5α-androstan-3-on
Z-17-(1')-Chlorethyliden-4-methyl-4-aza-5α-estran-3-on
Z-17-(1')-Bromethyliden-4-methyl-4-aza-5α-androstan-3-on
Z-17-(1')-Bromethyliden-4-methyl-4-aza-5α-estran-3-on

4. Verfahren zur Herstellung der Verbindungen nach Anspruch 1 mit R²⁰ = Fluoro-, Chloro- oder Bromo-,
R^{20a} = Wasserstoff
R⁴ = Me
R¹ und R² gemeinsam jeweils ein Wasserstoffatom,
**dadurch gekennzeichnet,**
**dass** man 17-Methylensteroide der Formel II, 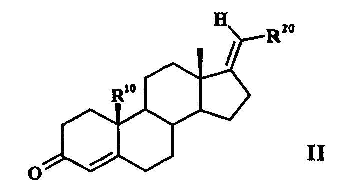 worin
R²⁰ für eine Fluoro-, Chloro- oder Bromo- steht und
R¹⁰ ein Wasserstoffatom oder eine Methylgruppe ist, in an sich bekannter Weise mit NalO₄ unter katalytischer Vermittlung von KMnO₄ in einem protischen Lösungsmittel zur 3,5-Seco-ketosäure umsetzt, diese mit NH₄OAc in Eisessig zum ungesättigten Lactam cyclisiert und mit HCOOH in DMF zum gesättigten Lactam der Formel III 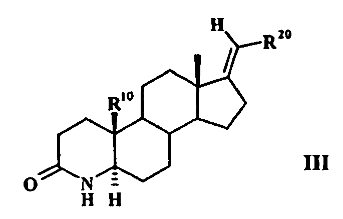 mit den Substituenten R²⁰ und R¹⁰ wie in Formel II, reduziert,
dann die Verbindungen der Formel III mit Mel und NaH zu den 4-methylsubstituierten Verbindungen der Formel IV 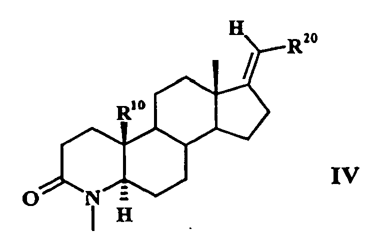 mit den Substituenten R²⁰ und R¹⁰ wie in Formel II umsetzt.

5. Verfahren zur Herstellung der Verbindungen nach Anspruch 1 mit R²⁰ = Fluoro-, Chloro- oder Bromo-
R^{20a} = Wasserstoff
R⁴ = Me
R¹ und R² gemeinsam jeweils ein Wasserstoffatom,
**dadurch gekennzeichnet,**
**dass** man 17-Methylensteroide der Formel II, 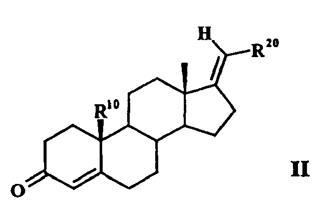 worin
R²⁰ für eine Fluoro-, Chloro- oder Bromo- steht und
R¹⁰ ein Wasserstoffatom oder eine Methylgruppe ist, wie im Anspruch 4 zum gesättigten Lactam der allgemeinen Formel III 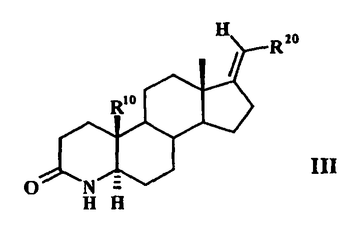 mit den Substituenten R²⁰ und R¹⁰ wie in Formel II reduziert
und in einer silylvermittelten Oxidation zu den dehydrierten Verbindungen der Formel V 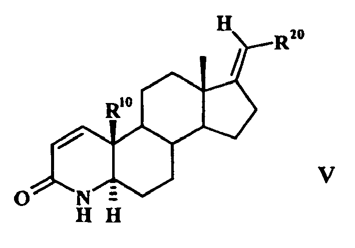 mit den Substituenten R²⁰ und R¹⁰ wie in Formel II, führt
und dann diese Verbindungen der Formel V mit
Mel und NaH zu den 4-methylsubstituierten Verbindungen der Formel VI 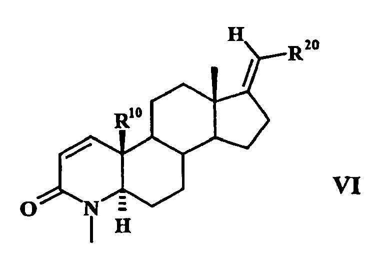 mit den Substituenten R²⁰ und R¹⁰ wie in Formel II,
umsetzt.

6. Verfahren zur Herstellung der Verbindungen nach Anspruch 1 mit R²⁰ = methyl
R^{20a} = Fluoro-, Chloro- oder Bromo-
R⁴ = Me oder
R¹ und R² gemeinsam jeweils ein Wasserstoffatom,
**dadurch gekennzeichnet,**
**dass** man 17-Methylensteroide der Formel VII, 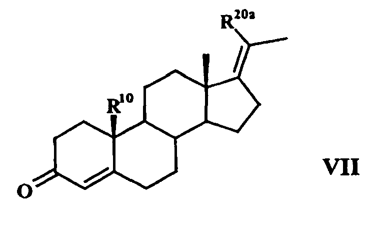 worin
R^{20a} für eine Fluoro-, Chloro- oder Bromo**-** steht
R²⁰ eine Methylgruppe und
R¹⁰ ein Wasserstoffatom oder eine Methylgruppe ist zu den Verbindungen der Formeln VIII und IX 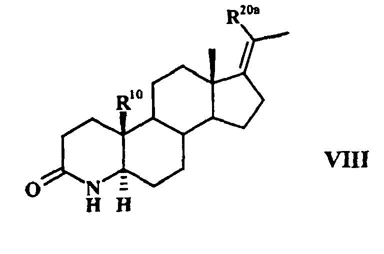 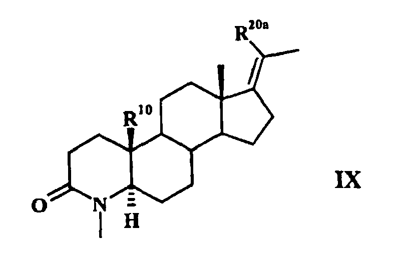 mit den Substituenten R^{20a} und R¹⁰ wie in Formel VII analog Anspruch 4
umsetzt.

7. Verfahren zur Herstellung der Verbindungen nach Anspruch 1 mit R²⁰ = Methyl
R^{20a} = Fluoro-, Chloro- oder Bromo-
R⁴ = Me oder
R¹ und R² gemeinsam jeweils ein Wasserstoffatom,
**dadurch gekennzeichnet,**
**dass** man 17-Methylensteroide der Formel VII, 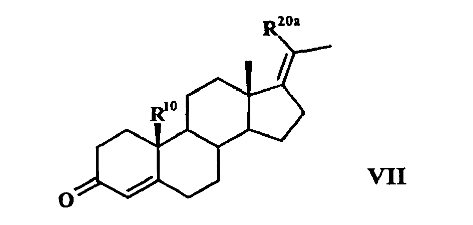 worin
R^{20a} für eine Fluoro-, Chloro- oder Bromo- steht
R²⁰ eine Methylgruppe und
R¹⁰ ein Wasserstoffatom oder eine Methylgruppe ist
zu den Verbindungen der Formeln X und XI 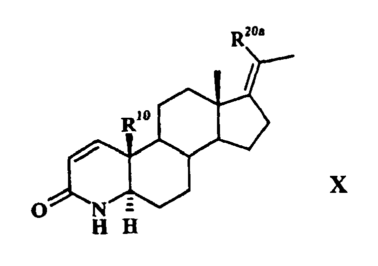 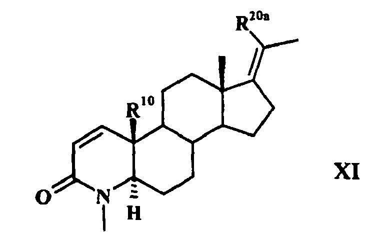 mit den Substituenten R^{20a} und R¹⁰ wie in Formel VII analog Anspruch 5
umsetzt.

8. Pharmazeutische Zusammensetzungen die mindestens ein 7-Methylen-4-azasteroid nach Anspruch 1 enthalten, gegebenenfalls zusammen mit pharmazeutisch verträglichen Hilfs- und Trägerstoffen.

9. Verwendung von mindestens einem 7-Methylen-4-azasteroid nach Anspruch 1 zur Herstellung eines pharmazeutischen Präparates zur Behandlung eines erhöhten Testosteronspiegels, einer erhöhten Umwandlung von Testosteron zu Dihydrotestosteron, der vorzeitigen Reifung und Eröffnung des Muttermundes, Prostataerkrankungen, Alopezie vom männtlichen Typ, Akne, Hirsutismus, gynäkologischen Krankheitsbilder wie prämenstruelles Syndrom und die vorzeitige Reifung der Cervix uteri.

## Claims

1. Halogen- and pseudohalogen-substituted 17-methylene-4-azasteroids of the general formula I 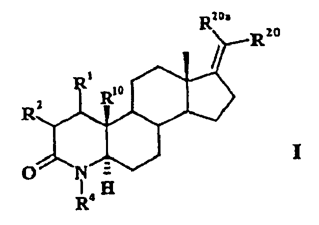 in which
R²⁰ and R^{20a} separately are fluoro, chloro, bromo, azido, cyano, thiocyanato, C₁₋₄-alkyl or hydroxy-C₁₋₄-alkyl,
and R^{20a} may further be a hydrogen atom,
R¹⁰ is a hydrogen atom or a methyl group
R⁴ is selected from a group consisting of hydrogen and C₁₋₄-alkyl, and
R¹ and R² together are in each case a hydrogen atom or an additional bond.

2. Halogen- and pseudohalogen-substituted 17-methylene-4-azasteroids according to Claim 1,
in which
R⁴ is H or Me and
R²⁰ and R^{20a} separately are F, Cl or Br.

3. 17-Methylene-4-azasteroids according to Claim 1, being
E-17-chloromethylene-4-aza-5α-estran-3-one
E-17-chloromethylene-4-aza-5α-androstan-3-one
E-17-chloromethylene-4-methyl-4-aza-5α-estran-3-one
E-17-chloromethylene-4-methyl-4-aza-5α-androstan-3-one
E-17-chloromethylene-4-aza-5α-estr-1-en-3-one
E-17-chloromethylene-4-aza-5α-androst-1-en-3-one
E-17-chloromethylene-4-methyl-4-aza-5α-androst-1-en-3-one
E-17-bromomethylene-4-methyl-4-aza-5α-estran-3-one
E-17-cyanomethylene-4-methyl-4-aza-5α-estran-3-one
Z-17-(1')-chloroethylidene-4-methyl-4-aza-5α-androstan-3-one
Z-17-(1')-chloroethylidene-4-methyl-4-aza-5α-estran-3-one
Z-17-(1')-bromoethylidene-4-methyl-4-aza-5α-androstan-3-one
Z-17-(1')-bromoethylidene-4-methyl-4-aza-5α-estran-3-one.

4. Process for preparing the compounds according to Claim 1
where R²⁰ is fluoro, chloro or bromo,
R^{20a} is hydrogen
R⁴ is Me and
R¹ and R² together are in each case a hydrogen atom,
**characterized in that**
17-methylene steroids of the formula II 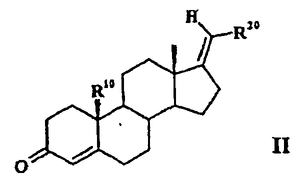 in which
R²⁰ is a fluoro, chloro or bromo and
R¹⁰ is a hydrogen atom or a methyl group are reacted in a manner known per se with NaIO₄ under catalytic mediation of KMnO₄ in a protic solvent to form the 3,5-seco-keto acid, said acid is cyclized with NH₄OAc in glacial acetic acid to form the unsaturated lactam and reduced with HCOOH in DMF to form the saturated lactam of the formula III 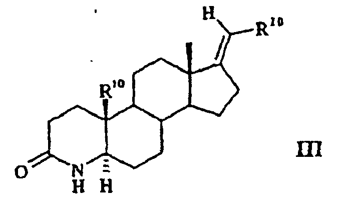 having the substituents R²⁰ and R¹⁰ as in formula II, then the compounds of the formula III are reacted with MeI and NaH to form the 4-methyl-substituted compounds of the formula IV 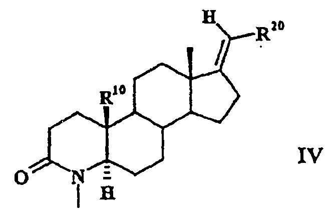 having the substituents R²⁰ and R¹⁰ as in formula II.

5. Process for preparing the compounds according to Claim 1 where R²⁰ is fluoro, chloro or bromo,
R^{20a} is hydrogen
R⁴ is Me and
R¹ and R² together are in each case a hydrogen atom, **characterized in that** 17-methylene steroids of the formula II 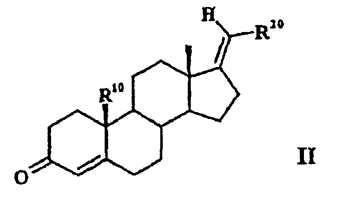 in which
R²⁰ is a fluoro, chloro or bromo and
R¹⁰ is a hydrogen atom or a methyl group are reduced as in Claim 4 to form the saturated lactam of the general formula III 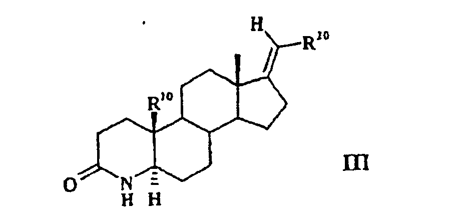 having the substituents R²⁰ and R¹⁰ as in formula II and said lactam is taken in a silyl-mediated oxidation to form the dehydrogenated compounds of the formula V 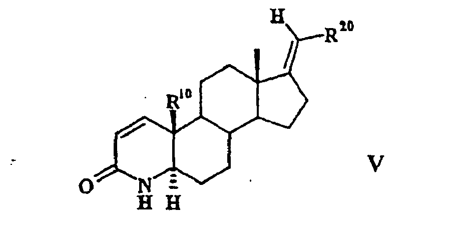 having the substituents R²⁰ and R¹⁰ as in formula II,
and then these compounds of the formula V are reacted with MeI and NaH to form the 4-methyl-substituted compounds of the formula VI 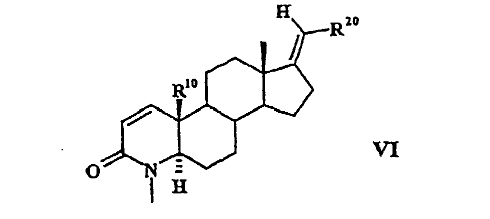 having the substituents R²⁰ and R¹⁰ as in formula II.

6. Process for preparing the compounds according to Claim 1 where R²⁰ is methyl,
R^{20a} is fluoro, chloro or bromo,
R⁴ is Me or H and
R¹ and R² together are in each case a hydrogen atom, **characterized in that** 17-methylene steroids of the formula VII 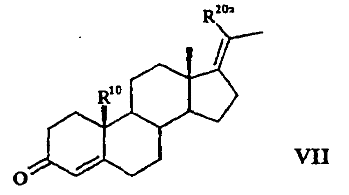 in which
R^{20a} is a fluoro, chloro or bromo
R²⁰ is a methyl group and
R¹⁰ is a hydrogen atom or a methyl group are reacted to form the compounds of the formulae VIII and IX 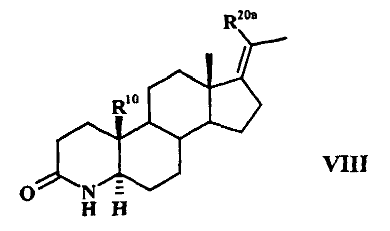 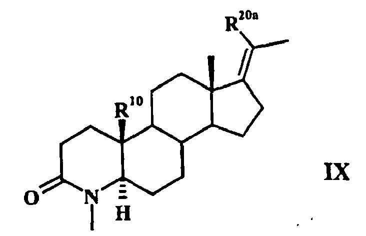 having the substituents R^{20a} and R¹⁰ as in formula VII, in analogy to Claim 4.

7. Process for preparing the compounds according to Claim 1 where R²⁰ is methyl,
R^{20a} is fluoro, chloro or bromo,
R⁴ is Me or H and
R¹ and R² together are in each case a hydrogen atom, **characterized in that** 17-methylene steroids of the formula VII 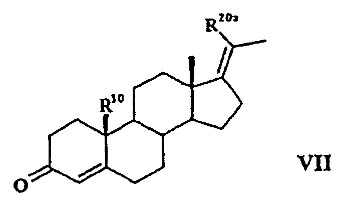 in which
R^{20a} is a fluoro, chloro or bromo,
R²⁰ is a methyl group and
R¹⁰ is a hydrogen atom or a methyl group
are reacted to form the compounds of the formulae X and XI 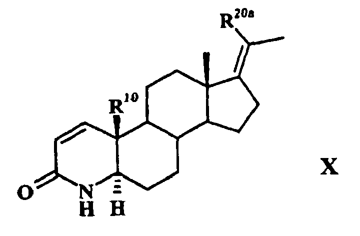 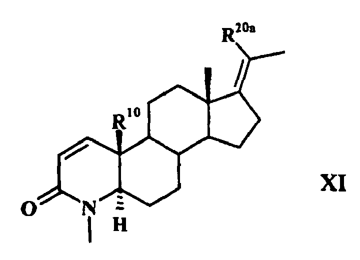 having the substituents R^{20a} and R¹⁰ as in formula VII, in analogy to Claim 5.

8. Pharmaceutical compositions which comprise at least one 17-methylene-4-azasteroid according to Claim 1, together if desired with pharmaceutically compatible auxiliary agents and carriers.

9. Use of at least one 17-methylene-4-azasteroid according to Claim 1 for manufacturing a pharmaceutical> product for treating an elevated testosterone level, an elevated conversion of testosterone to dihydrotestosterone, the premature maturation and opening of the os of uterus, prostate diseases, alopecia of the masculine type, acne, hirsutism, gynaecological clinical conditions such as premenstrual syndrome and the premature maturation of the cervix uteri.

## Revendications

1. 17-méthylène-4-azastéroïdes substitués par halogène et pseudohalogène de formule générale I 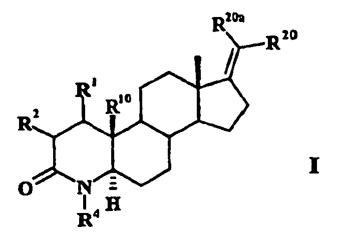 dans laquelle
R²⁰ et R^{20a} représentent, séparément, des groupes fluoro, chloro, bromo, azido, cyano, rhodano, alkyle en C₁ à C₄ ou hydroxyalkyle en C₁ à C₄, et R^{20a} peut en outre représenter un atome d'hydrogène
R¹⁰ représente un atome d'hydrogène ou un groupe méthyle,
R⁹ est choisi dans un groupe constitué par hydrogène et alkyle en C₁ à C₄,
R¹ et R² représentent à chaque fois ensemble un atome d'hydrogène ou une liaison supplémentaire.

2. 17-méthylène-4-azastéroïdes substitués par halogène et pseudohalogène selon la revendication 1,
dans laquelle
R⁴ signifie H, Me
R²⁰ et R^{20a} signifient séparément F, Cl, Br.

3. 17-méthylène-4-azastéroïdes selon la revendication 1, notamment
E-17-chlorométhylène-4-aza-5α-estran-3-one
E-17-chlorométhylène-4-aza-5α-androstan-3-one
E-17-chlorométhylène-4-méthyl-4-aza-5α-estran-3-one
E-17-chlorométhylène-4-méthyl-4-aza-5α-androstan-3-one
E-17-chlorométhylène-4-aza-5α-estr-1-én-3-one
E-17-chlorométhylène-4-aza-5α-androst-1-én-3-one
E-17-chlorométhylène-4-méthyl-4-aza-5α-androst-1-én-3-one
E-17-bromométhylène-4-méthyl-4-aza-5α-estran-3-one
E-17-cyanométhylène-4-méthyl-4-aza-5α-estran-3-one
Z-17-(1')-chloroéthylidène-4-méthyl-4-aza-5α-androstan-3-one
Z-17- (1')-chloroéthylidène-4-méthyl-4-aza-5α-estran-3-one
Z-17-(1')-bromoéthylidène-4-méthyl-4-aza-5α-androstan-3-one
Z-17-(1')-bromoéthylidène-4-méthyl-4-aza-5α-estran-3-one

4. Procédé pour la préparation des composés selon la revendication 1, avec
R²⁰ = fluoro, chloro ou bromo,
R^{20a} = hydrogène
R⁴ = Me
R¹ et R² représentant ensemble à chaque fois un atome d'hydrogène, **caractérisé en ce qu'**on transforme des 17-méthylènestéroïdes de formule II, 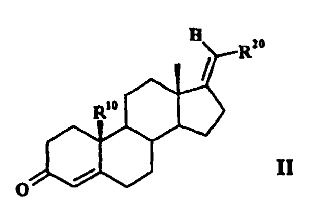 dans laquelle
R²⁰ représente fluoro, chloro ou bromo
et
R¹⁰ représente un atome d'hydrogène ou un groupe méthyle, de manière connue en soi avec NaIO₄ sous catalyse par KMnO₄ dans un solvant protique en acide 3,5-sécocétoïque, on cyclise celui-ci avec NH₄OAc dans de l'acide acétique glacial en lactame insaturé et puis on réduit avec HCOOH dans du DMF en lactame saturé de formule III 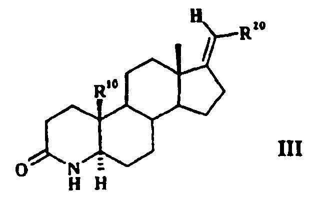 avec les substituants R²⁰ et R¹⁰ comme dans la formule II,
puis on transforme les composés de formule III avec MeI et NaH en composés substitués par 4-méthyle de formule IV 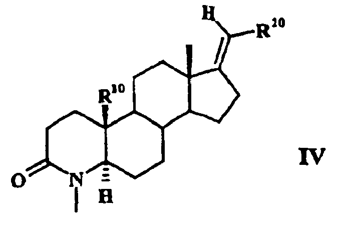 avec les substituants R²⁰ et R¹⁰ comme dans la formule II.

5. Procédé pour la préparation de composés selon la revendication 1 avec
R²⁰ = fluoro, chloro ou bromo
R^{20a} = hydrogène
R⁴ = Me
R¹ et R² représentent ensemble à chaque fois un atome d'hydrogène, **caractérisé en ce qu'**on réduit des 17-méthylènestéroïdes de formule II, 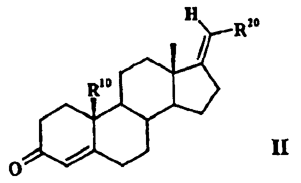 dans laquelle
R²⁰ représente fluoro, chloro ou bromo et
R¹⁰ représente un atome d'hydrogène ou un groupe méthyle comme dans la revendication 4 en lactame saturé de formule générale III 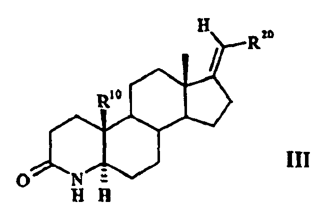 avec les substituants R²⁰ et R¹⁰ comme dans la formule II et on le transforme dans une oxydation avec intervention de silyle en composés déshydrogénés de formule V 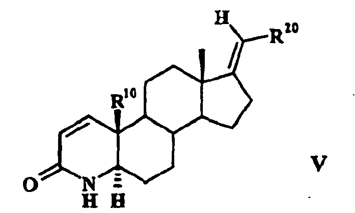 avec les substituants R²⁰ et R¹⁰ comme dans la formule II,
puis on transforme ces composés de formule V avec MeI et NaH en composés substitués par méthyle en 4ème position de formule VI 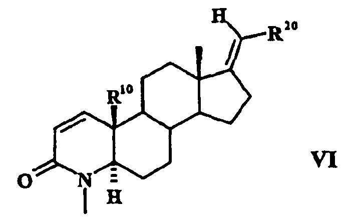 avec les substituants R²⁰ et R¹⁰ comme dans la formule II.

6. Procédé pour la préparation des composés selon la revendication 1
avec R²⁰ =méthyle
R^{20a} = fluoro, chloro ou bromo,
R⁹ = Me ou H
R¹ et R² représentent ensemble à chaque fois un atome d'hydrogène, **caractérisé en ce qu'**on transforme de manière analogue à la revendication 4 des 17-méthylènestéroïdes de formule VII. 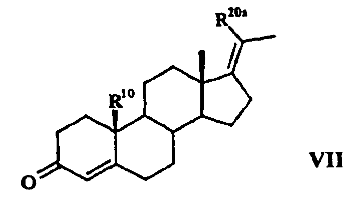 dans laquelle
R^{20a} représente fluoro, chloro ou bromo
R²⁰ représente un groupe méthyle et
R¹⁰ représente un atome d'hydrogène ou un groupe méthyle en composés de formules VIII et IX 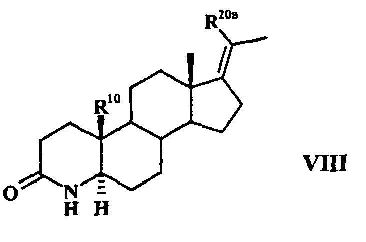 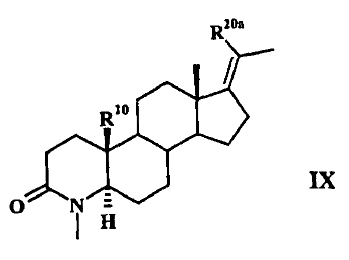 avec les substituants R^{20a} et R¹⁰ comme dans la formule VII.

7. Procédé pour la préparation des composés selon la revendication 1 avec
R²⁰ = méthyle
R^{20a} = fluoro, chloro ou bromo
R⁴ = Me ou H
R¹ et R² représentent ensemble à chaque fois un atome d'hydrogène, **caractérisé en ce qu'**on transforme de manière analogue à la revendication 5 des 17-méthylènestéroïdes de formule VII, 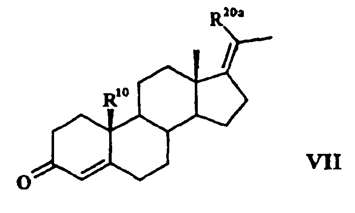 dans laquelle
R^{20a} représente fluoro, chloro ou bromo
R²⁰ représente un groupe méthyle et
R¹⁰ représente un atome d'hydrogène ou un groupe méthyle en composés de formules X et XI 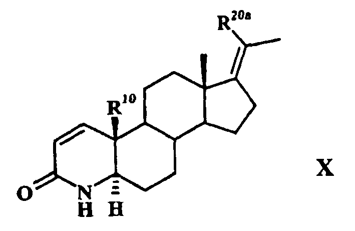 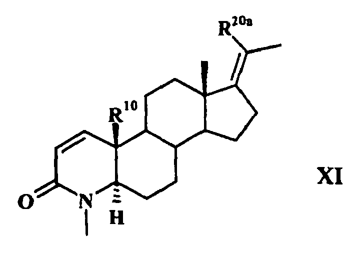 avec les substituants R^{20a} et R¹⁰ comme dans la formule VII.

8. Préparations pharmaceutiques qui contiennent au moins un 17-méthylène-4-azastéroïde selon la revendication 1, le cas échéant avec des adjuvants et des substances support pharmaceutiquement acceptables.

9. Utilisation d'au moins un 17-méthylène-4-azastéroïde selon la revendication 1 pour la préparation d'une préparation pharmaceutique destinée au traitement d'un niveau élevé de testostérone, d'une transformation élevée de testostérone en dihydrotestostérone, de la maturation prématurée et l'ouverture du col de l'utérus, des maladies de la prostate, de l'alopécie masculine, de l'acné, de l'hirsutisme, des maladies gynécologiques, telles que le syndrome prémenstruel et la maturation prématurée du col de l'utérus.
